# NEW EUROPEAN PATENT SPECIFICATION

(11) **EP 1 317 550 B2**
(45) Date of publication and mention of the opposition decision: **11.08.2010**
(45) Mention of the grant of the patent: 21.03.2007
(21) Application number: 01983438.1
(22) Date of filing: 16.08.2001
(51) Int. Cl.: C12N 15/54, C12N 1/21, C12P 13/08

(54) **NUCLEOTIDE SEQUENCES WHICH CODE FOR THE PPSA GENE**
FÜR DAS PPSA-GEN KODIERENDE NUKLEOTIDSEQUENZEN
SEQUENCES NUCLEOTIDES CODANT POUR LE GENE PPSA

(30) Priority: 13.09.2000 DE 10045497
(43) Date of publication of application: 11.06.2003
(73) Proprietor: Evonik Degussa GmbH, 45128 Essen (DE)
(72) Inventor: MÖCKEL, Bettina, 40597 Düsseldorf (DE); MARX, Achim, 33790 Halle (DE); BASTUCK, Christine, 33602 Bielefeld (DE); BUCHHOLZ, Michael, 33619 Bielefeld (DE); PFEFFERLE, Walter, 33790 Halle (Westf.) (DE)
(86) International application number: PCT/EP2001/009456
(87) International publication number: WO 2002/022829

(56) References cited:
- EP-A- 0 877 090
- EP-A- 1 108 790
- WO-A-01/00844
- NIERSBACH M ET AL: "CLONING AND NUCLEOTIDE SEQUENCE OF THE ESCHERICHIA COLI K-12 PPSA GENE, ENCODING PEP SYNTHASE" MOLECULAR AND GENERAL GENETICS, SPRINGER VERLAG, BERLIN, DE, vol. 231, 1992, pages 332-336, XP000942137 ISSN: 0026-8925
- MIKE S.M. JETTEN ET AL.: "Structural and functional analysis of pyruvate kinase from Corynebacterium glutamicum" APPLIED AND ENVIRONMENTAL MICROBIOLOGY, vol. 60, no. 7, 1994, pages 2501-2507, XP001073585
- MIKE S. JETTEN ET AL.: "Regulation of phospho(enol)-pyruvate- and oxaloacetate-converting enzymes in Corynebacterium gltamicum" APPLIED AND ENVIRONMENTAL BIOTECHNOLOGY, vol. 41, no. 1, 1994, pages 47-52, XP001073580
- DATABASE WPI Section Ch, Week 198346 Derwent Publications Ltd., London, GB; Class B05, AN 1983-817432 XP002198647 "Fermentative prepn. of L-Lysine" & JP 58 170487 A (AJINOMOTO KK), 7 October 1983 (1983-10-07)

## Description

### Field of the Invention

The invention provides nucleotide sequences from coryneform bacteria which code for the ppsA gene and a process for the fermentative preparation of L-Lysine using bacteria in which the ppsA gene is overexpressed.

### Prior Art

L-Amino acids, in particular L-lysine, are used in human medicine and in the pharmaceuticals industry, in the foodstuffs industry and very particularly in animal nutrition.

It is known that amino acids are prepared by fermentation from strains of coryneform bacteria, in particular Corynebacterium glutamicum. Because of their great importance, work is constantly being undertaken to improve the preparation processes. Improvements to the process can relate to fermentation measures, such as, for example, stirring and supply of oxygen, or the composition of the nutrient media, such as, for example, the sugar concentration during the fermentation, or the working up to the product form by, for example, ion exchange chromatography, or the intrinsic output properties of the microorganism itself.

Methods of mutagenes is, selection and mutant selection are used to improve the output properties of these microorganisms. Strains which are resistant to antimetabolites or are auxotrophic for metabolites of regulatory importance and produce amino acids are obtained in this manner.

Methods of the recombinant DNA technique have also been employed for some years for improving the strain of Corynebacterium strains which produce L-amino acid, by amplifying individual amino acid biosynthesis genes and investigating the effect on the amino acid production.

Molecular and General Genetics, 1992, Vol. 231, pages 332-336 (Niersbach et. al.) discloses the cloning and sequencing of E. coli K-12 ppsA gene encoding a phosphoenolpyruvate synthase. The given sequence is however different from the polynucleotide sequence of the present invention and no hint at a possible use in lysine production is given.

EP 0877090 discloses the use of E. coli phosphoenolpyruvate synthase gene to enhance aminoacid production in Corynebacterium. However, no hint is given herein that an overexpression of phosphoenolpyruvate synthase might bring about an increase in lysine production.

### Object of the Invention

The inventors had the object of providing new measures for improved fermentative preparation of L-lysine.

### Summary

When L-lysine or lysine are mentioned in the following, not only the bases but also the salts, such as e.g. lysine monohydrochloride or lysine sulfate, are meant by this.

The description provides an isolated polynucleotide from coryneform bacteria, comprising a polynucleotide sequence which codes for the ppsA gene, chosen from the group consisting of
a) polynucleotide which is identical to the extent of at least 70% to a polynucleotide which codes for a polypeptide which comprises the amino acid sequence of SEQ ID No. 2,
b) polynucleotide which codes for a polypeptide which comprises an amino acid sequence which is identical to the extent of at least 70% to the amino acid sequence of SEQ ID No. 2,
c) polynucleotide which is complementary to the polynucleotides of a) or b), and
d) polynucleotide comprising at least 15 successive nucleotides of the polynucleotide sequence of a), b) or c),
the polypeptide preferably having the activity of phosphoenol pyruvate synthase.

The description also provides the abovementioned polynucleotide, this preferably being a DNA which is capable of replication, comprising:
(i) the nucleotide sequence shown in SEQ ID No. 1, or
(ii) at least one sequence which corresponds to sequence (i) within the range of the degeneration of the genetic code, or
(iii) at least one sequence which hybridizes with the sequence complementary to sequence (i) or (ii), and optionally
(iv) sense mutations of neutral function in (i).

The invention provides
a polynucleotide, in particular DNA, which is capable of replication and comprises the nucleotide sequence as shown in SEQ ID No. 1;
a vector containing the polynucleotide according to the invention, in particular a shuttle vector or plasmid vector, and
coryneform bacteria which contain the vector or in which the ppsA gene is overexpressed.

The description also provides polynucleotides, which substantially comprise a polynucleotide sequence, which are obtainable by screening by means of hybridization of a corresponding gene library of a coryneform bacterium, which comprises the complete gene or parts thereof, with a probe which comprises the sequence of the polynucleotide according to the invention according to SEQ ID No.1 or a fragment thereof, and isolation of the polynucleotide sequence mentioned.

### Detailed Description

Polynucleotides which comprise the sequences according to the description are suitable as hybridization probes for RNA, cDNA and DNA, in order to isolate, in the full length, nucleic acids or polynucleotides or genes which code for phosphoenol pyruvate synthase or to isolate those nucleic acids or polynucleotides or genes which have a high similarity of sequence with that of the ppsA gene. They are also suitable for incorporation into so-called "arrays", "micro arrays" or "DNA chips" in order to detect and determine the corresponding polynucleotides.

Polynucleotides which comprise the sequences according to the description are furthermore suitable as primers with the aid of which DNA of genes which code for phosphoenol pyruvate synthase can be prepared by the polymerase chain reaction (PCR).

Such oligonucleotides which serve as probes or primers comprise at least 25, 26, 27, 28, 29 or 30, preferably at least 20, 21, 22, 23 or 24, very particularly preferably at least 15, 16, 17, 18 or 19 successive nucleotides. Oligonucleotides with a length of at least 31, 32, 33, 34, 35, 36, 37, 38, 39 or 40, or at least 41, 42, 43, 44, 45, 46, 47, 48, 49 or 50 nucleotides are also suitable. Oligonucleotides with a length of at least 100, 150, 200, 250 or 300 nucleotides are optionally also suitable. "Isolated" means separated out of its natural environment. "Polynucleotide" in general relates to polyribonucleotides and polydeoxyribonucleotides, it being possible for these to be non-modified RNA or DNA or modified RNA or DNA.

The polynucleotides according to the invention include a polynucleotide according to SEQ ID No. 1. The polynucleotides according to the description include a fragment prepared therefrom and also those which are at least 70% to 80%, preferably at least 81% to 85%, particularly preferably at least 86% to 90%, and very particularly preferably at least 91%, 93%, 95%, 97% or 99% identical to the polynucleotide according to SEQ ID No. 1 or a fragment prepared therefrom.

"Polypeptides" are understood as meaning peptides or proteins which comprise two or more amino acids bonded via peptide bonds.

The polypeptides according to the description include a polypeptide according to SEQ ID No. 2, in particular those with the biological activity of phosphoenol pyruvate synthase, and also those which are at least 70% to 80%, preferably at least 81% to 85%, particularly preferably at least 86% to 90%, and very particularly preferably at least 91%, 93%, 95%, 97% or 99% identical to the polypeptide according to SEQ ID No. 2 and have the activity mentioned.

The invention furthermore relates to a process for the fermentative preparation of L-lysine, using coryneform bacteria which in particular already produce L-lysine and in which the nucleotide sequences which code for the ppsA gene are over-expressed.

The term "enhancement" in this connection describes the increase in the intracellular activity of one or more enzymes in a microorganism which are coded by the corresponding DNA, for example by increasing the number of copies of the gene or genes, using a potent promoter or using a gene which codes for a corresponding enzyme having a high activity, and optionally combining these measures.

By enhancement measures, in particular over-expression, the activity or concentration of the corresponding protein is in general increased by at least 10%, 25%, 50%, 75%, 100%, 150%, 200%, 300%, 400% or 500%, up to a maximum of 1000% or 2000%, based on that of the wild-type protein or the activity or concentration of the protein in the starting microorganism.

The microorganisms which the present invention provides can produce L-lysine from glucose, sucrose, lactose, fructose, maltose, molasses, starch, cellulose or from glycerol and ethanol. They can be representatives of coryneform bacteria, in particular of the genus Corynebacterium. Of the genus Corynebacterium, there may be mentioned in particular the species Corynebacterium glutamicum, which is known among experts for its ability to produce L-lysine.

Suitable strains of the genus Corynebacterium, in particular of the species Corynebacterium glutamicum (C. glutamicum), are in particular the known wild-type strains
Corynebacterium glutamicum ATCC13032
Corynebacterium acetoglutamicum ATCC15806
Corynebacterium acetoacidophilum ATCC13870
Corynebacterium thermoaminogenes FERM BP-1539
Corynebacterium melassecola ATCC17965
Brevibacterium flavum ATCC14067
Brevibacterium lactofermentum ATCC13869 and
Brevibacterium divaricatum ATCC14020
and L- lysine-producing mutants or strains prepared therefrom.

The new ppsA gene from C. glutamicum (SEQ ID NO:1) which codes for the enzyme phosphoenol pyruvate synthase (EC 2.7.9.2) has been isolated.

To isolate the ppsA gene or also other genes of C. glutamicum, a gene library of this microorganism is first set up in Escherichia coli (E. coli). The setting up of gene libraries is described in generally known textbooks and handbooks. The textbook by Winnacker: Gene und Klone, Eine Einführung in die Gentechnologie (Verlag Chemie, Weinheim, Germany, 1990), or the handbook by Sambrook et al.: Molecular Cloning, A Laboratory Manual (Cold Spring Harbor Laboratory Press, 1989) may be mentioned as an example. A well-known gene library is that of the E. coli K-12 strain W3110 set up in λ vectors by Kohara et al. (Cell 50, 495 -508 (1987)). Bathe et al. (Molecular and General Genetics, 252:255-265, 1996) describe a gene library of C. glutamicum ATCC13032, which was set up with the aid of the cosmid vector SuperCos I (Wahl et al., 1987, Proceedings of the National Academy of Sciences USA, 84:2160-2164) in the E. coli K-12 strain NM554 (Raleigh et al., 1988, Nucleic Acids Research 16:1563-1575).

Börmann et al. (Molecular Microbiology 6(3), 317-326) (1992)) in turn describe a gene library of C. glutamicum ATCC13032 using the cosmid pHC79 (Hohn and Collins, Gene 11, 291-298 (1980)).

To prepare a gene library of C. glutamicum in E. coli it is also possible to use plasmids such as pBR322 (Bolivar, Life Sciences, 25, 807-818 (1979)) or pUC9 (Vieira et al., 1982, Gene, 19:259-268). Suitable hosts are, in particular, those E. coli strains which are restriction- and recombination-defective. An example of these is the strain DH5αmcr, which has been described by Grant et al. (Proceedings of the National Academy of Sciences USA, 87 (1990) 4645-4649). The long DNA fragments cloned with the aid of cosmids can in turn be subcloned in the usual vectors suitable for sequencing and then sequenced, as is described e.g. by Sanger et al. (Proceedings of the National Academy of Sciences of the United States of America, 74:5463-5467, 1977).

The resulting DNA sequences can then be investigated with known algorithms or sequence analysis programs, such as e.g. that of Staden (Nucleic Acids Research 14, 217-232(1986)), that of Marck (Nucleic Acids Research 16, 1829-1836 (1988)) or the GCG program of Butler (Methods of Biochemical Analysis 39, 74-97 (1998)).

The new DNA sequence of C. glutamicum which codes for the ppsA gene and which, as SEQ ID No. 1, is a constituent of the present invention has been found. The amino acid sequence of the corresponding protein has furthermore been derived from the present DNA sequence by the methods described above. The resulting amino acid sequence of the ppsA gene product is shown in SEQ ID No. 2.

Coding DNA sequences which result from SEQ ID No. 1 by the degeneracy of the genetic code are also a constituent of the description. In the same way, DNA sequences which hybridize with SEQ ID No. 1 or parts of SEQ ID No. 1 are a constituent of the description. Conservative amino acid exchanges, such as e.g. exchange of glycine for alanine or of aspartic acid for glutamic acid in proteins, are furthermore known among experts as "sense mutations" which do not lead to a fundamental change in the activity of the protein, i.e. are of neutral function. It is furthermore known that changes on the N and/or C terminus of a protein cannot substantially impair or can even stabilize the function thereof. Information in this context can be found by the expert, inter alia, in Ben-Bassat et al. (Journal of Bacteriology 169:751-757 (1987)), in O'Regan et al. (Gene 77:237-251 (1989)), in Sahin-Toth et al. (Protein Sciences 3:240-247 (1994)), in Hochuli et al. (Bio/Technology 6:1321-1325 (1988)) and in known textbooks of genetics and molecular biology. Amino acid sequences which result in a corresponding manner from SEQ ID No. 2 are also a constituent of the description.

In the same way, DNA sequences which hybridize with SEQ ID No. 1 or parts of SEQ ID No. 1 are a constituent of the description. Finally, DNA sequences which are prepared by the polymerase chain reaction (PCR) using primers which result from SEQ ID No. 1 are a constituent of the description. Such oligonucleotides typically have a length of at least 15 nucleotides.

Instructions for identifying DNA sequences by means of hybridization can be found by the expert, inter alia, in the handbook "The DIG System Users Guide for Filter Hybridization" from Boehringer Mannheim GmbH (Mannheim, Germany, 1993) and in Liebl et al. (International Journal of Systematic Bacteriology (1991) 41: 255-260). The hybridization takes place under stringent conditions, that is to say only hybrids in which the probe and target sequence, i. e. the polynucleotides treated with the probe, are at least 70% identical are formed. It is known that the stringency of the hybridization, including the washing steps, is influenced or determined by varying the buffer composition, the temperature and the salt concentration. The hybridization reaction is preferably carried out under a relatively low stringency compared with the washing steps (Hybaid Hybridisation Guide, Hybaid Limited, Teddington, UK, 1996).

A 5x SSC buffer at a temperature of approx. 50°C - 68°C, for example, can be employed for the hybridization reaction. Probes can also hybridize here with polynucleotides which are less than 70% identical to the sequence of the probe. Such hybrids are less stable and are removed by washing under stringent conditions. This can be achieved, for example, by lowering the salt concentration to 2x SSC and optionally subsequently 0.5x SSC (The DIG System User's Guide for Filter Hybridisation, Boehringer Mannheim, Mannheim, Germany, 1995) a temperature of approx. 50°C - 68°C being established. It is optionally possible to lower the salt concentration to 0.1x SSC. Polynucleotide fragments which are, for example, at least 70% or at least 80% or at least 90% to 95% identical to the sequence of the probe employed can be isolated by increasing the hybridization temperature stepwise from 50°C to 68°C in steps of approx. 1 - 2°C. Further instructions on hybridization are obtainable on the market in the form of so-called kits (e.g. DIG Easy Hyb from Roche Diagnostics GmbH, Mannheim, Germany, Catalogue No. 1603558).

Instructions for amplification of DNA sequences with the aid of the polymerase chain reaction (PCR) can be found by the expert, inter alia, in the handbook by Gait: Oligonucleotide Synthesis: A Practical Approach (IRL Press, Oxford, UK, 1984) and in Newton and Graham: PCR (Spektrum Akademischer Verlag, Heidelberg, Germany, 1994).

It has been found that coryneform bacteria produce L-lysine in an improved manner after over-expression of the ppsA gene.

To achieve an over-expression, the number of copies of the corresponding genes can be increased, or the promoter and regulation region or the ribosome binding site upstream of the structural gene can be mutated. Expression cassettes which are incorporated upstream of the structural gene act in the same way. By inducible promoters, it is additionally possible to increase the expression in the course of fermentative L-lysine production. The expression is likewise improved by measures to prolong the life of the m-RNA. Furthermore, the enzyme activity is also increased by preventing the degradation of the enzyme protein. The genes or gene constructs can either be present in plasmids with a varying number of copies, or can be integrated and amplified in the chromosome. Alternatively, an over-expression of the genes in question can furthermore be achieved by changing the composition of the media and the culture procedure.

Instructions in this context can be found by the expert, inter alia, in Martin et al. (Bio/Technology 5, 137-146 (1987)), in Guerrero et al. (Gene 138, 35-41 (1994)), Tsuchiya and Morinaga (Bio/Technology 6, 428-430 (1988)), in Eikmanns et al. (Gene 102, 93-98 (1991)), in EP 0 472 869, in US 4,601,893, in Schwarzer and Pühler (Bio/Technology 9, 84-87 (1991), in Reinscheid et al. (Applied and Environmental Microbiology 60, 126-132 (1994)), in LaBarre et al. (Journal of Bacteriology 175, 1001-1007 (1993)), in WO 96/15246, in Malumbres et al. (Gene 134, 15 - 24 (1993)), in JP-A-10-229891, in Jensen and Hammer (Biotechnology and Bioengineering 58, 191-195 (1998)), in Makrides (Microbiological Reviews 60:512-538 (1996)) and in known textbooks of genetics and molecular biology.

For enhancement, by way of example the ppsA gene according to the invention was over-expressed with the aid of episomal plasmids. Suitable plasmids are those which are replicated in coryneform bacteria. Numerous known plasmid vectors, such as e.g. pZ1 (Menkel et al., Applied and Environmental Microbiology (1989) 64: 549-554), pEKEx1 (Eikmanns et al., Gene 102:93-98 (1991)) or pHS2-1 (Sonnen et al., Gene 107:69-74 (1991)) are based on the cryptic plasmids pHM1519, pBL1 or pGA1. Other plasmid vectors, such as e.g. those based on pCG4 (US-A 4,489,160), or pNG2 (Serwold-Davis et al., FEMS Microbiology Letters 66, 119-124 (1990)), or pAG1 (US-A 5,158,891), can be used in the same manner.

Plasmid vectors which are furthermore suitable are also those with the aid of which the process of gene amplification by integration into the chromosome can be used, as has been described, for example, by Reinscheid et al. (Applied and Environmental Microbiology 60, 126-132 (1994)) for duplication or amplification of the hom-thrB operon. In this method, the complete gene is cloned in a plasmid vector which can replicate in a host (typically E. coli), but not in C. glutamicum. Possible vectors are, for example, pSUP301 (Simon et al., Bio/Technology 1, 784-791 (1983)), pK18mob or pK19mob (Schäfer et al., Gene 145, 69-73 (1994)), pGEM-T (Promega corporation, Madison, WI, USA), pCR2.1-TOPO (Shuman (1994). Journal of Biological Chemistry 269:32678-84; US-A 5,487,993), pCR®Blunt (Invitrogen, Groningen, Holland; Bernard et al., Journal of Molecular Biology, 234: 534-541 (1993)), pEM1 (Schrumpf et al, 1991, Journal of Bacteriology 173:4510-4516) or pBGS8 (Spratt et al.,1986, Gene 41: 337-342). The plasmid vector which contains the gene to be amplified is then transferred into the desired strain of C. glutamicum by conjugation or transformation. The method of conjugation is described, for example, by Schäfer et al. (Applied and Environmental Microbiology 60, 756-759 (1994)). Methods for transformation are described, for example, by Thierbach et al. (Applied Microbiology and Biotechnology 29, 356-362 (1988)), Dunican and Shivnan (Bio/Technology 7, 1067-1070 (1989)) and Tauch et al. (FEMS Microbiological Letters 123, 343-347 (1994)). After homologous recombination by means of a "cross over" event, the resulting strain contains at least two copies of the gene in question.

In addition, it may be advantageous for the production of L-lysine to over-express one or more enzymes of the particular biosynthesis pathway, of glycolysis, of anaplerosis, of the citric acid cycle, of the pentose phosphate cycle, of amino acid export and optionally regulatory proteins, in addition to the ppsA gene.

Thus, for the preparation of L-amino acids, in addition to enhancement of the ppsA gene, one or more genes chosen from the group consisting of
- the dapA gene which codes for dihydrodipicolinate synthase (EP-B 0 197 335),
- the gap gene which codes for glyceraldehyde 3-phosphate dehydrogenase (Eikmanns (1992), Journal of Bacteriology 174:6076-6086),
- the tpi gene which codes for triose phosphate isomerase (Eikmanns (1992), Journal of Bacteriology 174:6076-6086),
- the pgk gene which codes for 3-phosphoglycerate kinase (Eikmanns (1992), Journal of Bacteriology 174:6076-6086),
- the zwf gene which codes for glucose 6-phosphate dehydrogenase (JP-A-09224661),
- the pyc gene which codes for pyruvate carboxylase (DE-A-198 31 609),
- the mqo gene which codes for malate-quinone oxidoreductase (Molenaar et al., European Journal of Biochemistry 254, 395-403 (1998)),
- the lysC gene which codes for a feed-back resistant aspartate kinase (Accession No.P26512),
- the lysE gene which codes for lysine export (DE-A-195 48 222),
- the hom gene which codes for homoserine dehydrogenase (EP-A 0131171),
- the ilvA gene which codes for threonine dehydratase (Möckel et al., Journal of Bacteriology (1992) 8065- • 8072)) or the ilvA(Fbr) allele which codes for a "feed back resistant" threonine dehydratase (Möckel et al., (1994) Molecular Microbiology 13: 833-842),
- the ilvBN gene which codes for acetohydroxy-acid synthase (EP-B 0356739),
- the ilvD gene which codes for dihydroxy-acid dehydratase (Sahm and Eggeling (1999) Applied and Environmental Microbiology 65: 1973-1979),
- the zwa1 gene which codes for the Zwa1 protein (DE: 19959328.0, DSM 13115),
can be over-expressed.

It may furthermore be advantageous for the production of L-lysine, in addition to the enhancement of the ppsA gene, for one or more genes chosen from the group consisting of
- the pck gene which codes for phosphoenol pyruvate carboxykinase (DE 199 50 409.1; DSM 13047),
- the pgi gene which codes for glucose 6-phosphate isomerase (US 09/396,478; DSM 12969),
- the poxB gene which codes for pyruvate oxidase (DE: 1995 1975.7; DSM 13114),
- the zwa2 gene which codes for the Zwa2 protein (DE: 19959327.2, DSM 13113)
to be attenuated, in particular for the expression thereof to be reduced.

The term "attenuation" in this connection describes the reduction or elimination of the intracellular activity of one or more enzymes (proteins) in a microorganism which are coded by the corresponding DNA, for example by using a weak promoter or using a gene or allele which codes for a corresponding enzyme with a low activity or inactivates the corresponding gene or enzyme (protein), and optionally combining these measures.

By attenuation measures, the activity or concentration of the corresponding protein is in general reduced to 0 to 75%, 0 to 50%, 0 to 25%, 0 to 10% or 0 to 5% of the activity or concentration of the wild-type protein or of the activity or concentration of the protein in the starting microorganism.

In addition to over-expression of the ppsA gene it may furthermore be advantageous for the production of amino acids to eliminate undesirable side reactions (Nakayama: "Breeding of Amino Acid Producing Micro-organisms", in: Overproduction of Microbial Products, Krumphanzl, Sikyta, Vanek (eds.), Academic Press, London, UK, 1982).

The invention also provides the microorganisms prepared according to the invention, and these can be cultured continuously or discontinuously in the batch process (batch culture) or in the fed batch (feed process) or repeated fed batch process (repetitive feed process) for the purpose of production of amino acids. A summary of known culture methods is described in the textbook by Chmiel (Bioprozesstechnik 1. Einführung in die Bioverfahrenstechnik (Gustav Fischer Verlag, Stuttgart, 1991)) or in the textbook by Storhas (Bioreaktoren und periphere Einrichtungen (Vieweg Verlag, Braunschweig/Wiesbaden, 1994)).

The culture medium to be used must meet the requirements of the particular strains in a suitable manner. Descriptions of culture media for various microorganisms are contained in the handbook "Manual of Methods for General Bacteriology" of the American Society for Bacteriology (Washington D.C., USA, 1981).

Sugars and carbohydrates, such as e.g. glucose, sucrose, lactose, fructose, maltose, molasses, starch and cellulose, oils and fats, such as e.g. soya oil, sunflower oil, groundnut oil and coconut fat, fatty acids, such as e.g. palmitic acid, stearic acid and linoleic acid, alcohols, such as e.g. glycerol and ethanol, and organic acids, such as e.g. acetic acid, can be used as the source of carbon. These substances can be used individually or as a mixture.

Organic nitrogen-containing compounds, such as peptones, yeast extract, meat extract, malt extract, corn steep liquor, soya bean flour and urea, or inorganic compounds, such as ammonium sulfate, ammonium chloride, ammonium phosphate, ammonium carbonate and ammonium nitrate, can be used as the source of nitrogen. The sources of nitrogen can be used individually or as a mixture.

Phosphoric acid, potassium dihydrogen phosphate or dipotassium hydrogen phosphate or the corresponding sodium-containing salts can be used as the source of phosphorus. The culture medium must furthermore comprise salts of metals, such as e. g. magnesium sulfate or iron sulfate, which are necessary for growth. Finally, essential growth substances, such as amino acids and vitamins, can be employed in addition to the above-mentioned substances. Suitable precursors can moreover be added to the culture medium. The starting substances mentioned can be added to the culture in the form of a single batch, or can be fed in during the culture in a suitable manner.

Basic compounds, such as sodium hydroxide, potassium hydroxide, ammonia or aqueous ammonia, or acid compounds, such as phosphoric acid or sulfuric acid, can be employed in a suitable manner to control the pH of the culture. Antifoams, such as e.g. fatty acid polyglycol esters, can be employed to control the development of foam. Suitable substances having a selective action, such as e.g. antibiotics, can be added to the medium to maintain the stability of plasmids. To maintain aerobic conditions, oxygen or oxygen-containing gas mixtures, such as e.g. air, are introduced into the culture. The temperature of the culture is usually 20°C to 45°C, and preferably 25°C to 40°C. Culturing is continued until a maximum of the desired product has formed. This target is usually reached within 10 hours to 160 hours.

Methods for the determination of L-lysine acids are known from the prior art. The analysis can thus be carried out, for example, as described by Spackman et al. (Analytical Chemistry, 30, (1958), 1190) by ion exchange chromatography with subsequent ninhydrin derivation, or it can be carried out by reversed phase HPLC, for example as described by Lindroth et al. (Analytical Chemistry (1979) 51: 1167-1174).

The process according to the invention is used for fermentative preparation of L-lysine.

The present invention is explained in more detail in the following with the aid of embodiment examples.

The isolation of plasmid DNA from Escherichia coli and all techniques of restriction, Klenow and alkaline phosphatase treatment were carried out by the method of Sambrook et al. (Molecular Cloning. A Laboratory Manual (1989) Cold Spring Harbor Laboratory Press, Cold Spring Harbor, NY, USA). Methods for transformation of Escherichia coli are also described in this handbook.

The composition of the usual nutrient media, such as LB or TY medium, can also be found in the handbook by Sambrook et al.

### Example 1

Preparation of a genomic cosmid gene library from Corynebacterium glutamicum ATCC 13032

Chromosomal DNA from Corynebacterium glutamicum ATCC 13032 was isolated as described by Tauch et al. (1995, Plasmid 33:168-179) and partly cleaved with the restriction enzyme Sau3AI (Amersham Pharmacia, Freiburg, Germany, Product Description Sau3AI, Code no. 27-0913-02). The DNA fragments were dephosphorylated with shrimp alkaline phosphatase (Roche Diagnostics GmbH, Mannheim, Germany, Product Description SAP, Code no. 1758250). The DNA of the cosmid vector SuperCos1 (Wahl et al. (1987) Proceedings of the National Academy of Sciences USA 84:2160-2164), obtained from Stratagene (La Jolla, USA, Product Description SuperCos1 Cosmid Vector Kit, Code no. 251301) was cleaved with the restriction enzyme XbaI (Amersham Pharmacia, Freiburg, Germany, Product Description XbaI, Code no. 27-0948-02) and likewise dephosphorylated with shrimp alkaline phosphatase.

The cosmid DNA was then cleaved with the restriction enzyme BamHI (Amersham Pharmacia, Freiburg, Germany, Product Description BamHI, Code no. 27-0868-04). The cosmid DNA treated in this manner was mixed with the treated ATCC13032 DNA and the batch was treated with T4 DNA ligase (Amersham Pharmacia, Freiburg, Germany, Product Description T4-DNA-Ligase, Code no.27-0870-04). The ligation mixture was then packed in phages with the aid of Gigapack II XL Packing Extract (Stratagene, La Jolla, USA, Product Description Gigapack II XL Packing Extract, Code no. 200217).

For infection of the E. coli strain NM554 (Raleigh et al. 1988, Nucleic Acid Research 16:1563-1575) the cells were taken up in 10 mM MgSO₄ and mixed with an aliquot of the phage suspension. The infection and titering of the cosmid library were carried out as described by Sambrook et al. (1989, Molecular Cloning: A laboratory Manual, Cold Spring Harbor), the cells being plated out on LB agar (Lennox, 1955, Virology, 1:190) with 100 mg/l ampicillin. After incubation overnight at 37°C, recombinant individual clones were selected.

### Example 2

### Isolation and sequencing of the ppsA gene

The cosmid DNA of an individual colony was isolated with the Qiaprep Spin Miniprep Kit (Product No. 27106, Qiagen, Hilden, Germany) in accordance with the manufacturer's instructions and partly cleaved with the restriction enzyme Sau3AI (Amersham Pharmacia, Freiburg, Germany, Product Description Sau3AI, Product No. 27-0913-02). The DNA fragments were dephosphorylated with shrimp alkaline phosphatase (Roche Diagnostics GmbH, Mannheim, Germany, Product Description SAP, Product No. 1758250). After separation by gel electrophoresis, the cosmid fragments in the size range of 1500 to 2000 bp were isolated with the QiaExII Gel Extraction Kit (Product No. 20021, Qiagen, Hilden, Germany).

The DNA of the sequencing vector pZero-1, obtained from Invitrogen (Groningen, Holland, Product Description Zero Background Cloning Kit, Product No. K2500-01), was cleaved with the restriction enzyme BamHI (Amersham Pharmacia, Freiburg, Germany, Product Description BamHI, Product No. 27-0868-04). The ligation of the cosmid fragments in the sequencing vector pZero-1 was carried out as described by Sambrook et al. (1989, Molecular Cloning: A Laboratory Manual, Cold Spring Harbor), the DNA mixture being incubated overnight with T4 ligase (Pharmacia Biotech, Freiburg, Germany). This ligation mixture was then electroporated (Tauch et al. 1994, FEMS Microbiol Letters, 123:343-7) into the E. coli strain DH5αMCR (Grant, 1990, Proceedings of the National Academy of Sciences U.S.A., 87:4645-4649) and plated out on LB agar (Lennox, 1955, Virology, 1:190) with 50 mg/l zeocin.

The plasmid preparation of the recombinant clones was carried out with the Biorobot 9600 (Product No. 900200, Qiagen, Hilden, Germany). The sequencing was carried out by the dideoxy chain termination method of Sanger et al. (1977, Proceedings of the National Academy of Sciences U.S.A., 74:5463-5467) with modifications according to Zimmermann et al. (1990, Nucleic Acids Research, 18:1067). The "RR dRhodamin Terminator Cycle Sequencing Kit" from PE Applied Biosystems (Product No. 403044, Weiterstadt, Germany) was used. The separation by gel electrophoresis and analysis of the sequencing reaction were carried out in a "Rotiphoresis NF Acrylamide/Bisacrylamide" Gel (29:1) (Product No. A124.1, Roth, Karlsruhe, Germany) with the "ABI Prism 377" sequencer from PE Applied Biosystems (Weiterstadt, Germany).

The raw sequence data obtained were then processed using the Staden program package (1986, Nucleic Acids Research, 14:217-231) version 97-0. The individual sequences of the pZero1 derivatives were assembled to a continuous contig. The computer-assisted coding region analysis was prepared with the XNIP program (Staden, 1986, Nucleic Acids Research, 14:217-231).

The resulting nucleotide sequence is shown in SEQ ID No. 1. Analysis of the nucleotide sequence showed an open reading frame of 1095 base pairs, which was called the ppsA gene. The ppsA gene codes for a protein of 364 amino acids.

## Claims

1. Process for the preparation of L-lysine, wherein the following steps are carried out:
a) fermentation of a coryneform bacterium comprising overexpression of polynucleotide encoding a polypeptide which is at least 90% identical to the amino acid sequence of SEQ ID NO:2 and having the activity of a phosphoenol pyruvate synthase in a medium suitable for the formation of said L-lysine,
b) concentration of said L-lysine in the medium or in the cells of said coryneform bacterium, and
c) isolation of said L-lysine.

2. The process of claim 1, wherein said polypeptide is at least 95% identical to the amino acid sequence of SEQ ID NO:2.

3. The process of claim 1 or 2, wherein said polypeptide comprises the amino acid sequence of SEQ ID NO:2.

4. The process of claim 1,2 or 3, wherein said polynucleotide comprises the nucleotide sequence of nucleotides 200 to 1291 of SEQ ID NO:1.

5. The process of claim 1, 2 or 3, wherein said polynucleotide comprises the nucleotide sequence of SEQ ID NO:1 carrying sense mutations of neutral function.

6. The process of claim 1, 2, or 3, wherein said polynucleotide comprises the nucleotide sequence of SEQ ID NO:1.

7. The process of any of the claims 1 to 7, wherein said coryneform bacterium is of the genus Corynebacterium.

8. The process of claim 7, wherein said coryneform bacterium is of the species Corynebacterium glutamicum.

9. The process of any of the claims 1 to 8, wherein said overexpression is achieved by increasing the copy number of said polyuncleotide.

10. The process of any of claims 1-8 wherein said overexpression is achieved by linking a promoter to said polynucleotide.

11. The process of claim 9 wherein said increasing of the copy number is achieved by using a plasmid.

12. The process of claim 9 wherein said increasing of the copy number is achieved by integrating said copies into the chromosome.

13. The process of any of the claims 1 to 12, wherein one or more genes selected from the group consisting of
13.1 the dapA gene which codes for dihydrodipicolinate synthase,
13.2 the gap gene which codes for glyceraldehyde 3-phosphate dehydrogenase,
13.3 the tpi gene which codes for triose phosphate isomerase,
13.4 the pgk gene which codes for 3-phosphoglycerate kinase,
13.5 the zwf gene which codes for glucose 6-phosphate dehydrogenase,
13.6 the pyc gene which codes for pyruvate carboxylase,
13.7 the mqo gene which codes for malate-quinone oxidoreductase,
13.8 the lysC gene which codes for a feed-back resistant aspartate kinase,
13.9 the lysE gene which codes for lysine export,
13.10 the hom gene which codes for homoserine dehydrogenase
13.11 the ilvA gene which codes for threonine dehydratase or the ilvA(Fbr) allele which codes for a feed back resistant threonine dehydratase,
13.12 the ilvBN gene which codes for acetohydroxy-acid synthase,
13.13 the ilvD gene which codes for dihydroxy-acid dehydratase,
13.14 the zwa1 gene which codes for the Zwa1 protein,
is or are overexpressed.

14. The process of any of the claims 1 to 12, wherein one or more genes selected from the group consisting of
14.1 the pck gene which codes for phosphoenol pyruvate carboxykinase,
14.2 the pgi gene which codes for glucose 6-phosphate isomerase,
14.3 the poxB gene which codes for pyruvate oxidase
14.4 the zwa2 gene which codes for the Zwa2 protein
is or are attenuated.

15. A coryneform bacterium comprising a polynucleotide, wherein said polynucleotide comprises the nucleotide sequence of SEQ ID NO: 1 carrying sense mutations of neutral function.

16. A coryneform bacterium comprising a polynucleotide, wherein said polynucleotide comprises the nucleotide sequence of SEQ ID NO: 1.

17. A vector comprising an isolated polynucleotide wherein said polynucleotide comprises the nucleotide sequence of SEQ ID NO: 1.

18. A coryneform bacterium or a bacterium of the species Escherichia coli transformed with the vector of claim 17.

## Patentansprüche

1. Verfahren zur Herstellung von L-Lysin, bei dem man folgende Schritte durchführt:
a) Fermentation eines coryneformen Bakteriums, enthaltend Überexprimierung eines für ein Polypeptid, das zu mindestens 90% mit der Aminosäuresequenz von SEQ ID NO: 2 identisch ist und die Aktivität einer Phosphoenolpyruvat-Synthase aufweist, kodierendes Polynukleotid in einem zur Bildung des L-Lysins geeigneten Medium,
b) Anreicherung des L-Lysins im Medium oder in den Zellen des coryneformen Bakteriums und
c) Isolieren des L-Lysins.

2. Verfahren nach Anspruch 1, bei dem das Polypeptid zu mindestens 95% mit der Aminosäuresequenz von SEQ ID NO: 2 identisch ist.

3. Verfahren nach Anspruch 1 oder 2, bei dem das Polypeptid die Aminosäuresequenz von SEQ ID NO: 2 enthält.

4. Verfahren nach Anspruch 1, 2 oder 3, bei dem das Polynukleotid die Nukleotidsequenz der Nukleotide 200 bis 1291 von SEQ ID NO: 1 enthält.

5. Verfahren nach Anspruch 1, 2 oder 3, bei dem das Polynukleotid die funktionsneutrale Sinnmutationen tragende Nukleotidsequenz von SEQ ID NO: 1 enthält.

6. Verfahren nach Anspruch 1, 2 oder 3, bei dem das Polynukleotid die Nukleotidsequenz von SEQ ID NO: 1 enthält.

7. Verfahren nach einem der Ansprüche 1 bis 7, bei dem das coryneforme Bakterium zur Gattung Corynebacterium gehört.

8. Verfahren nach Anspruch 7, bei dem das coryneforme Bakterium zur Art Corynebacterium glutamicum gehört.

9. Verfahren nach einem der Ansprüche 1 bis 8, bei dem die Überexprimierung durch Erhöhen der Kopienzahl des Polynukleotids erfolgt.

10. Verfahren nach einem der Ansprüche 1 bis 8, bei dem die Überexprimierung durch Verknüpfen eines Promotors mit dem Polynukleotid erfolgt.

11. Verfahren nach Anspruch 9, bei dem man die Kopienzahl durch Verwendung eines Plasmids erhöht.

12. Verfahren nach Anspruch 9, bei dem man die Kopienzahl durch Einbau der Kopien in das Chromosom erhöht.

13. Verfahren nach einem der Ansprüche 1 bis 12, bei dem man eines oder mehrere der Gene, ausgewählt aus der Gruppe
13.1 das für die Dihydrodipicolinat-Synthase kodierende Gen dapA,
13.2 das für die Glyceraldehyd-3-Phosphat-Dehydrogenase kodierende Gen gap,
13.3 das für die Triosephosphat-Isomerase kodierende Gen tpi,
13.4 das für die 3-Phosphoglycerat-Kinase kodierende Gen pgk,
13.5 das für die Glukose-6-Phosphat-Dehydrogenase kodierende Gen zwf,
13.6 das für die Pyruvat-Carboxylase kodierende Gen pyc,
13.7 das für die Malat-Chinon-Oxidoreduktase kodierende Gen mqo,
13.8 das für eine feed-back-resistente Aspartatkinase kodierende Gen lysC,
13.9 das für den Lysin-Export kodierende Gen lysE,
13.10das für die Homoserin-Dehydrogenase kodierende Gen hom,
13.11das für die Threonin-Dehydratase kodierende Gen ilvA oder das für eine feed-back-resistente Threonin-Dehydratase kodierende Allel ilvA(Fbr),
13.12das für die Acetohydroxysäure-Synthase kodierende Gen ilvBN,
13.13das für die Dihydroxysäuredehydratase kodierende Gen ilvD,
13.14das für das Zwal-Protein kodierende Gen zwa1, überexprimiert.

14. Verfahren nach einem der Ansprüche 1 bis 12, bei dem man eines oder mehrere der Gene, ausgewählt aus der Gruppe
14.1 das für die Phosphoenolpyruvat-Carboxykinase kodierende Gen pck,
14.2 das für die Glukose-6-Phosphat-Isomerase kodierende Gen pgi,
14.3 das für die Pyruvat-Oxidase kodierende Gen poxB,
14.4 das für das Zwa2-Protein kodierende Gen zwa2, abschwächt.

15. Coryneformes Bakterium, enthaltend ein Polynukleotid, bei dem das Polynukleotid die funktionsneutrale Sinnmutationen tragende Nukleotidsequenz von SEQ ID NO: 1 enthält.

16. Coryneformes Bakterium, enthaltend ein Polynukleotid, bei dem das Polynukleotid die Nukleotidsequenz von SEQ ID NO: 1 enthält.

17. Vektor, enthaltend ein isoliertes Polynukleotid, bei dem das Polynukleotid die Nukleotidsequenz von SEQ ID NO: 1 enthält.

18. Coryneformes Bakterium oder ein Bakterium der Art Escherichia coli, transformiert mit dem Vektor gemäß Anspruch 17.

## Revendications

1. Processus pour la préparation de L-lysine, dans lequel les étapes suivantes sont exécutées :
a) la fermentation d'une bactérie corynéforme, comprenant la surexpression d'un polynucléotide codant pour un polypeptide qui est identique, au moins à 90%, à la séquence d'acides aminés de la SEQ ID n°: 2 et ayant l'activité d'une phosphoénol pyruvate synthase dans un milieu convenant à la formation de ladite L-lysine,
b) la concentration de ladite L-lysine dans le milieu ou dans les cellules de ladite bactérie corynéforme et
c) l'isolement de ladite L-lysine.

2. Processus selon la revendication 1, dans lequel ledit polypeptide est identique, au moins à 95%, à la séquence d'acides aminés de la SEQ ID n°: 2.

3. Processus selon la revendication 1 ou la 2, dans lequel ledit polypeptide comprend la séquence d'acides aminés de la SEQ ID n° : 2.

4. Processus selon les revendications 1, 2 ou 3, dans lequel ledit polynucléotide comprend la séquence nucléotidique des nucléotides 200 à 1291 de la SEQ ID n° : 1.

5. Processus selon les revendications 1, 2 ou 3, dans lequel ledit polynucléotide comprend la séquence nucléotidique de la SEQ ID n° : 1 transportant des mutations sens à fonction neutre.

6. Processus selon les revendications 1, 2 ou 3, dans lequel ledit polynucléotide comprend la séquence nucléotidique de la SEQ ID n° : 1.

7. Processus selon l'une quelconque des revendications 1 à 7, dans lequel ladite bactérie corynéforme est du genre Corynebacterium.

8. Processus selon la revendication 7, dans lequel ladite bactérie corynéforme est de l'espèce Corynebacterium glutamicum.

9. Processus selon l'une quelconque des revendications 1 à 8, dans lequel ladite surexpression est accomplie en augmentant le nombre de copies dudit polynucléotide.

10. Processus selon l'une quelconque des revendications 1 à 8, dans lequel ladite surexpression est accomplie en liant un promoteur au dit polynucléotide.

11. Processus selon la revendication 9, dans lequel ladite augmentation du nombre de copies est accomplie en utilisant un plasmide.

12. Processus selon la revendication 9, dans lequel ladite augmentation du nombre de copies est accomplie en intégrant lesdites copies dans le chromosome.

13. Processus selon l'une quelconque des revendications 1 à 12, dans lequel un ou plusieurs gène(s), choisis dans le groupe composé :
13.1 du gène dapA, qui code pour une dihydrodipicolinate synthase,
13.2 du gène gap, qui code pour une glycéraldéhyde 3-phosphate déshydrogénase,
13.3 du gène tpi, qui code pour une triose phosphate isomérase,
13.4 du gène pgk, qui code pour une 3-phosphoglycérate kinase,
13.5 du gène zwf, qui code pour une glucose 6-phosphate déshydrogénase,
13.6 du gène pyc, qui code pour une pyruvate carboxylase,
13.7 du gène mqo, qui code pour une malate-quinone oxydoréductase,
13.8 du gène lysC, qui code pour une aspartate kinase résistante au rétrocontrôle,
13.9 du gène lysE, qui code pour une exportation de la lysine,
13.10 du gène hom, qui code pour une homosérine déshydrogénase,
13.11 du gène ilvA, qui code pour une thréonine déshydratase, ou l'allèle ilvA(Fbr), qui code pour une thréonine déshydratase résistante au rétrocontrôle,
13.12 du gène ilvBN, qui code pour une acétohydroxy-acide synthase,
13.13 du gène ilvD, qui code pour une dihydroxy-acide déshydratase,
13.14 du gène zwa1, qui code pour la protéine Zwa1, est ou sont surexprimé(s).

14. Processus selon l'une quelconque des revendications 1 à 12, dans lequel un ou plusieurs gène(s), choisis dans le groupe composé :
14.1 du gène pck, qui code pour une phosphoénol pyruvate carboxykinase,
14.2 du gène pgi, qui code pour une glucose 6-phosphate isomérase,
14.3 du gène poxB, qui code pour une pyruvate oxydase,
14.4 du gène zwa2, qui code pour la protéine Zwa2, est ou sont atténué (s) .

15. Bactérie corynéforme comprenant un polynucléotide, dans laquelle ledit polynucléotide comprend la séquence nucléotidique de la SEQ ID n° : 1 transportant des mutations sens à fonction neutre.

16. Bactérie corynéforme comprenant un polynucléotide, dans laquelle ledit polynucléotide comprend la séquence nucléotidique de la SEQ ID n° : 1.

17. Vecteur comprenant un polynucléotide isolé, dans lequel ledit polynucléotide comprend la séquence nucléotidique de la SEQ ID n° : 1.

18. Bactérie corynéforme ou bactérie de l'espèce Escherichia coli transformée avec le vecteur selon la revendication 17.


### SEQUENCE LISTING

<110> Degussa AG
<120> Nucleotide sequences which code for the ppsA gene
<130> 000175 BT
<140>
   <141>
<160> 2
<170> PatentIn Ver. 2.1
<210> 1
   <211> 1494
   <212> DNA
   <213> Corynebacterium glutamicum
<220>
   <221> CDS
   <222> (200)..(1291)
   <223> ppsA gene
<400> 1
   cggcatccac tattgcctag gaaacatgct ggccaaactt caagccaaga tctgtctcga 60
   ggaagtcacc aggcttgttc cttccctgca cttggttgcg gacaaagcta tcgggttccg 120
   ggagaacctc tccttccgcg tccccacttc tgttcccgtg acttggaacg cttaacgctt 180
   tattaaataa ggagacacc atg acc aac agt ttg aac atc ccg ttt gtc cag  232
                        Met Thr Asn Ser Leu Asn Ile Pro Phe Val Gln
                          1               5                  10
   cgc ttc gat gaa ggc ctg gat cct gtt cta gaa gta ctc ggt ggc aag   280
   Arg Phe Asp Glu Gly Leu Asp Pro Val Leu Glu Val Leu Gly Gly Lys
               15                  20                  25
   ggc gct tca cta gtc acc atg aca gat gct gga atg ccc gtt cca cct   328
   Gly Ala Ser Leu Val Thr Met Thr Asp Ala Gly Met Pro Val Pro Pro
           30                  35                  40
   gga ttt gtg gtc act act gcc agc ttt gat gaa ttc atc cgt gaa gca   376
   Gly Phe Val Val Thr Thr Ala Ser Phe Asp Glu Phe Ile Arg Glu Ala
       45                  50                  55
   ggg gtt gct gaa cac atc gat aaa ttc cta aac gat ctc gat gca gaa   424
   Gly Val Ala Glu His Ile Asp Lys Phe Leu Asn Asp Leu Asp Ala Glu
   60                  65                  70                  75
   gat gtt aag gaa gtg gat cga gtt tct gcg atc atc cgc gat gag ctg   472
   Asp Val Lys Glu Val Asp Arg Val Ser Ala Ile Ile Arg Asp Glu Leu
                   80                  85                  90
   tgc agt ctt gac gtt cca gag aat gct cgt ttc gca gtg cac cag gct   520
   Cys Ser Leu Asp Val Pro Glu Asn Ala Arg Phe Ala Val His Gln Ala
               95                  100                 105
   tat cgc gat ctc atg gaa cga tgc ggt ggc gac gtc ccg gtt gct gtc      568
   Tyr Arg Asp Leu Met Glu Arg Cys Gly Gly Asp Val Pro Val Ala Val
           110                 115                 120
   cgg tca tcg gcc act gcc gaa gat ctg ccc gat gct tcc ttc gca ggg      616
   Arg Ser Ser Ala Thr Ala Glu Asp Leu Pro Asp Ala Ser Phe Ala Gly
       125                 130                 135
   caa cag gac acc tat ctg tgg caa gtc ggt ttg agc gct gtc act gaa      664
   Gln Gln Asp Thr Tyr Leu Trp Gln Val Gly Leu Ser Ala Val Thr Glu
   140                 145                 150                 155
   cac atc cgt aaa tgc tgg gct tcg ctg ttc act tcc cgt gcc att atc      712
   His Ile Arg Lys Cys Trp Ala Ser Leu Phe Thr Ser Arg Ala Ile Ile
                   160                 165                 170
   tac cgt ctg aaa aac aac atc ccc aat gag ggc ctc tcc atg gcg gta      760
   Tyr Arg Leu Lys Asn Asn Ile Pro Asn Glu Gly Leu Ser Met Ala Val
               175                 180                 185
   gtt gtt caa aaa atg gtc aac tct cgt gtc gca ggc gtg gca atc act      808
   Val Val Gln Lys Met Val Asn Ser Arg Val Ala Gly Val Ala Ile Thr
           190                 195                 200
   atg aat cct tcc aac ggc gac cgc tcg aag atc acc atc gat tcc tca      856
   Met Asn Pro Ser Asn Gly Asp Arg Ser Lys Ile Thr Ile Asp Ser Ser
       205                 210                 215
   tgg ggt gtt ggt gaa atg gtg gtc tca ggt gaa gtg aca cca gac aat      904
   Trp Gly Val Gly Glu Met Val Val Ser Gly Glu Val Thr Pro Asp Asn
   220                 225                 230                 235
   atc ttg ctg gac aag atc acg ctg cag gtt gtc tcc gaa cac att gga      952
   Ile Leu Leu Asp Lys Ile Thr Leu Gln Val Val Ser Glu His Ile Gly
                   240                 245                 250
   agc aaa cac gct gaa ctc atc ccc gat gcc acc agt gga agc ctc gtg      1000
   Ser Lys His Ala Glu Leu Ile Pro Asp Ala Thr Ser Gly Ser Leu Val
               255                 260                 265
   gaa aag ccc gtt gat gaa gaa cgc gca aac cgc cgc agt ctg act gat      1048
   Glu Lys Pro Val Asp Glu Glu Arg Ala Asn Arg Arg Ser Leu Thr Asp
           270                 275                 280
   gag gaa atg ctc gct gtg gca caa atg gct aag cgt gca gaa aaa cac      1096
   Glu Glu Met Leu Ala Val Ala Gln Met Ala Lys Arg Ala Glu Lys His
       285                 290                 295
   tac aag tgc cca caa gat atc gaa tgg gcg ctg gac gct gat ctg cca      1144
   Tyr Lys Cys Pro Gln Asp Ile Glu Trp Ala Leu Asp Ala Asp Leu Pro
   300                 305                 310                 315
   gat gga gaa aac ctt ctg tta ttg caa tcc cgc ccg gaa act atc cac      1192
   Asp Gly Glu Asn Leu Leu Leu Leu Gln Ser Arg Pro Glu Thr Ile His
                   320                 325                 330
   tcc aac ggt gtg aag aag gaa acc cca act ccg cag gct gcc aaa acc      1240
   Ser Asn Gly Val Lys Lys Glu Thr Pro Thr Pro Gln Ala Ala Lys Thr
               335                 340                 345
   ata ggc acc ttc gat ttc agc tca atc acc gtc gca atg acc ggc acg    1288
   Ile Gly Thr Phe Asp Phe Ser Ser Ile Thr Val Ala Met Thr Gly Thr
           350                 355                 360
   aag taaaaccacc gcatcttttc gtcgaaaagc atctaaaagg agtttgacca         1341
   Lys
   tggctaataa atctttcccc aagccctccg atcttccagt gcccaagggc gctgaaggtt 1401
   gggaagatct gtacccgtac tacctcgttt tccaagacaa gctcatggat caagagaatg 1461
   agaaattctg gttctgcgat tcacagcact ggc                               1494
<210> 2
   <211> 364
   <212> PRT
   <213> Corynebacterium glutamicum
<400> 2
   Met Thr Asn Ser Leu Asn Ile Pro Phe Val Gln Arg Phe Asp Glu Gly
    1               5                  10                  15
   Leu Asp Pro Val Leu Glu Val Leu Gly Gly Lys Gly Ala Ser Leu Val
               20                  25                  30
   Thr Met Thr Asp Ala Gly Met Pro Val Pro Pro Gly Phe Val Val Thr
           35                  40                  45
   Thr Ala Ser Phe Asp Glu Phe Ile Arg Glu Ala Gly Val Ala Glu His
       50                  55                  60
   Ile Asp Lys Phe Leu Asn Asp Leu Asp Ala Glu Asp Val Lys Glu Val
   65                  70                  75                  80
   Asp Arg Val Ser Ala Ile Ile Arg Asp Glu Leu Cys Ser Leu Asp Val
                   85                  90                  95
   Pro Glu Asn Ala Arg Phe Ala Val His Gln Ala Tyr Arg Asp Leu Met
               100                 105                 110
   Glu Arg Cys Gly Gly Asp Val Pro Val Ala Val Arg Ser Ser Ala Thr
           115                 120                 125
   Ala Glu Asp Leu Pro Asp Ala Ser Phe Ala Gly Gln Gln Asp Thr Tyr
       130                 135                 140
   Leu Trp Gln Val Gly Leu Ser Ala Val Thr Glu His Ile Arg Lys Cys
   145                 150                 155                 160
   Trp Ala Ser Leu Phe Thr Ser Arg Ala Ile Ile Tyr Arg Leu Lys Asn
                   165                 170                 175
   Asn Ile Pro Asn Glu Gly Leu Ser Met Ala Val Val Val Gln Lys Met
               180                 185                 190
   Val Asn Ser Arg Val Ala Gly Val Ala Ile Thr Met Asn Pro Ser Asn
           195                 200                 205
   Gly Asp Arg Ser Lys Ile Thr Ile Asp Ser Ser Trp Gly Val Gly Glu
       210                 215                 220
   Met Val Val Ser Gly Glu Val Thr Pro Asp Asn Ile Leu Leu Asp Lys
   225                 230                 235                     240
   Ile Thr Leu Gln Val Val Ser Glu His Ile Gly Ser Lys His Ala Glu
                   245                 250                     255
   Leu Ile Pro Asp Ala Thr Ser Gly Ser Leu Val Glu Lys Pro Val Asp
               260                 265                 270
   Glu Glu Arg Ala Asn Arg Arg Ser Leu Thr Asp Glu Glu Met Leu Ala
           275                 280                 285
   Val Ala Gln Met Ala Lys Arg Ala Glu Lys His Tyr Lys Cys Pro Gln
       290                 295                 300
   Asp Ile Glu Trp Ala Leu Asp Ala Asp Leu Pro Asp Gly Glu Asn Leu
   305                 310                 315                     320
   Leu Leu Leu Gln Ser Arg Pro Glu Thr Ile His Ser Asn Gly Val Lys
                   325                 330                     335
   Lys Glu Thr Pro Thr Pro Gln Ala Ala Lys Thr Ile Gly Thr Phe Asp
               340                 345                 350
   Phe Ser Ser Ile Thr Val Ala Met Thr Gly Thr Lys
           355                 360